# EUROPEAN PATENT APPLICATION

(11) **EP 4 215 056 A1**
(43) Date of publication of application: **26.07.2023**
(21) Application number: 23155009.6
(22) Date of filing: 16.04.2019
(51) Int. Cl.: A23L 2/58, A23D 7/005, A23D 7/01, C09B 61/00, C09B 47/00, C09B 67/00, A23L 29/10, A23L 5/44, A23L 5/47, C09B 67/08

(54) **PIGMENT-LOADED SOLID LIPID NANOPARTICLES**

(30) Priority: 23.04.2018 EP 18168715
(62) Divisional of application: 19716932.9
(71) Applicant: Oterra A/S, 2970 Hørsholm (DK); Københavns Universitet, 1165 København K (DK)
(72) Inventor: SCHJOERRING-THYSSEN, Jakob, 2970 Hørsholm (DK); KOEHLER, Klaus, 2970 Hørsholm (DK); ANDERSEN, Mogens Larsen, 2970 Hørsholm (DK); OLSEN, Karsten, 2970 Hørsholm (DK)
(74) Representative: Willnegger, Eva

(57) **Abstract**

Solid lipid nanoparticles (SLNs) and suspensions thereof in an aqueous phase are provided, in which the SLNs have high content of oil-soluble pigment and high stability. A food product, such as a beverage, comprising said SLNs is also provided.

## Description

### TECHNICAL FIELD

Solid lipid nanoparticles (SLNs) are provided, in which said nanoparticles comprise an oil-soluble pigment and a high-melting lipid. A suspension comprising such SLNs in an aqueous phase is also provided.

### BACKGROUND

Natural pigments such as carotenoids and chlorophylls are generally unstable in the presence of light, heat or oxygen. Additionally, such pigments tend to be insoluble in water, and stabilizing matrix formulations are typically required when such pigments are used in aqueous environments, e.g. in foodstuffs.

Solid lipid nanoparticles (SLNs) are formulations based on small, spherical particles. SLNs contain an active substance with the lipid core, and one or more surfactants are typically used to promote stability and dispersibility in an aqueous phase.

SLNs which contain carotenes are known from e.g. Helgason et al. J. Agric. Food. Chem. 2009, 57, 8033-8040 and Gutiérrez et al. Trends in Food Science & Technology, 32 (2013) 73-83.

The light-sensitive nature of pigments means that, increased pigment concentration in an SLN leads to increased uptake and concentration of light energy within the SLN. This can - in turn - lead to degradation of the pigment and/or SLN, see e.g. *Helgason et al.* ibid. Known pigment-containing SLN formulations typically have low pigment concentration.

A particular problem for pigment-containing SLNs is therefore to achieve increased pigment concentration in the SLN, while maintaining stability. Increasing the pigment concentration allows savings in the manufacture and use of the SLNs, as less SLN can be used to achieve the same pigmenting effect in e.g. a foodstuff. Additionally, the SLN itself should form a stable aqueous suspension in an aqueous environment.

The present technology aims to address such problems in pigment-containing SLNs.

### SUMMARY OF THE INVENTION

So, in a first aspect, a solid lipid nanoparticle (SLN) is provided which comprises:
a. a core comprising:
   i. a lipid having a melting point above 40°C, and
   ii. an oil-soluble pigment
b. a dual surfactant system comprising
   i. a polysorbate, and
   ii. a phospholipid
wherein the ratio of polysorbate:phospholipid (i:ii) in said dual surfactant system is between 10:1 and 1:10 and the amount of pigment is 5-40% w/w of said SLN.

In a further aspect, a suspension comprising said solid lipid nanoparticles (SLNs) as defined herein in an aqueous phase is provided.

In yet a further aspect, a method for producing a suspension of solid lipid nanoparticles (SLNs) as described herein is provided, said method comprising the steps of:
A. Providing a liquid oil phase comprising (a) a lipid having a melting point above 40°C; and (b) an oil-soluble pigment, and heating said liquid oil phase so as to dissolve the oil-soluble pigment;
B. Providing an aqueous phase comprising a dual surfactant system (c), which comprises (i) a polysorbate, and (ii) a phospholipid, such that the ratio of polysorbate:phospholipid (i:ii) is between 10:1 and 1:10;
C. Mixing said liquid oil phase from step A. with said aqueous phase from step B. in a high shear mixer to create an emulsion;
D. Passing the emulsified mixture from step C through a homogenizer;
E. Cooling the homogenized mixture from step D, thereby providing a suspension of solid lipid nanoparticles (SLNs).

A suspension is provided comprising first solid lipid nanoparticles (SLN-1) and second solid lipid nanoparticles (SLN-2) in a single aqueous phase, wherein each of SLN-1 and SLN-2 are as described herein, and wherein the oil-soluble pigments in each of SLN-1 and SLN-2 are different.

A food product, preferably a beverage, is provided, comprising solid lipid nanoparticles as described herein.

The use of solid lipid nanoparticles as described herein as a colorant in a food product, such as a beverage is also provided.

### DETAILED DISCLOSURE

Solid lipid nanoparticles (SLNs) are provided, which have a particular use in food products such as beverages.

In the context of the present invention SLNs are solid at 25°C, and preferably have a particle size of less than 300nm. Particle size being defined as the Z-average measured by dynamic light scattering.

In a first aspect, a solid lipid nanoparticle (SLN) comprising (a) a core and (b) a dual surfactant system is provided. It has been discovered that SLNs with particular compositions (especially those with a particular dual surfactant system) have improved stability over other compositions.

The core (a) of said SLN comprises (i) a lipid having a melting point above 40°C, and (ii) an oil-soluble pigment. During production, a mixture of the lipid and the oil-soluble pigment are warmed together, so that the pigment dissolves in the lipid. The pigment thus becomes homogeneously blended in the lipid. The pigment remains non-crystalline upon solidification of the lipid.

The lipid is the major component of the SLN core, and is typically present in an amount of between 30 and 70% w/w of said SLN, preferably between 40 and 65% w/w, more preferably 45 and 65% w/w. The lipid is typically a saturated lipid and may be but are not limited to a fully saturated triglyceride, such as e.g. fully hydrogenated sunflower oil, fully hydrogenated rapeseed oil fully hydrogenated palm oil or fully hydrogenated soybean oil. The lipid preferably has a melting point above 40°C, such as above 45°C, such as above 50°C. Further the lipid preferably has a melting point below 100°C, such as below 98°C, such as below 95°C, such as below 85°C, such as below 75°C.

The oil-soluble pigment is the minor component of the SLN core and is present in an amount of between 5 and 40% w/w of said SLN, such as between 5 and 25% w/w, preferably between 5 and 20% w/w. Oil-soluble pigments useful in the present technology are naturally-occurring oil-soluble pigments, such as e.g. carotenoids or chlorophylls. Particular carotenoids include α-carotene, β-carotene, lycopene, lutein, bixin and norbixin. Of these, β-carotene is preferred.

The dual surfactant system (b) of said SLN is located at the surface of the SLN, and acts both to stabilize the SLN and to ensure its miscibility in aqueous systems. The dual surfactant system (b) is present in an amount of between 20 and 70% w/w, 25 and 75% w/w, 25 and 50% w/w, suitably between 30 and 40% w/w of said SLN.

The dual surfactant system (b) comprises (i) a polysorbate, and (ii) a phospholipid, and the ratio of polysorbate:phospholipid (i:ii) in said dual surfactant system is between 10:1 and 1:10. In a particular embodiment the ratio is between 5:1 and 1:5, such as 4:1 and 1:4, such as 2:1 and 1:2. Suitably, the polysorbate and phospholipid components are present in equal amounts, or an excess of polysorbate is present. Suitably, therefore, the ratio of polysorbate:phospholipid (i:ii) in said dual surfactant system is between 10:1 and 1:1. In a particular embodiment the ratio is between 5:1 and 1:1, such as 4:1 and 1:1, such as 2:1 and 1:1. As an alternative, the ratio of polysorbate:phospholipid (i :ii) in said dual surfactant system is between 1:1 and 1:10. In a particular embodiment the ratio is between 1:1 and 1:5, such as 1:1 and 1:2.

Polysorbates are emulsifiers based on ethoxylated sorbitan esterified with fatty acids. Typically said polysorbate is polysorbate 80, although other polysorbates such as polysorbate 60, polysorbate 40 and polysorbate 20 may be used.

The phospholipid according to one particular embodiment is selected from sunflower lecithin, soy bean lecithin, cotton seed lecithin, rape seed lecithin or egg yolk lecithin.

The SLNs additionally comprise an antioxidant, suitably a tocopherol such as α-tocopherol. The antioxidant may be present in an amount of between 1-10 % (such as around 5%) w/w of said SLN. Such SLNs have improved stability over other SLNs.

The SLN according to this technology suitably have a mean particle size, measured by dynamic light scattering, of 100-150nm. Particle size is determined according to the method defined in the examples, and is provided in terms of Z-average (average particle size) and PDI (polydispersity index).

In a second aspect, a suspension comprising solid lipid nanoparticles (SLNs) as described herein in an aqueous phase is provided.

It has been found that it is possible to prepare a suspension with high lipid and pigment content with the particular SLNs of the invention. In order to use SLN suspensions as food coloring additives, it is necessary to have a high concentration of pigment. If the concentration is <1%, the required addition of suspension to a product to obtain a coloring property would be so high that issues with off-flavor and composition changes in the final product i.e. a food application would arise. With the present invention it is possible to reach a pigment content above 1% w/w in the suspension, and thereby make it interesting for the color industry. At the same time, increasing the net content of pigment in the suspension also limits the net amount of product needed to be produced and transported making production easier and more economically feasible.

Suitably, the suspension consists of SLNs as defined herein in an aqueous phase; i.e. no other components are present.

In terms of the amounts of each component, the suspension may comprise:
i. a lipid having a melting point above 40°C, in an amount of 10 to 35% w/w of the total suspension; and
ii. an oil-soluble pigment, in an amount of 1 to 20% w/w of the total suspension;
iii. a dual surfactant system, in an amount of 1 to 25% w/w of the total suspension.

The lipid having a melting point above 40°C is typically present in an amount of 10 to 35% preferably 10 to 20% w/w, more preferably 14 to 18% w/w of the total suspension.

The phospholipid is typically present in an amount of 0.2 to 10%, 0.5 to 8%, 1 to 7%, 1 to 6% w/w, preferably 1 to 5% w/w of the suspension.

The polysorbate is typically present in an amount of 1 to 20% w/w, such as 2 to 15% such as 5 to 10%.

The oil-soluble pigment is typically present in an amount of 1 to 20% w/w, 1 to 10% preferably 1 to 5% w/w of said suspension.

The amounts of each component in the suspension are given in w/w % of the total suspension. The remaining weight of the suspension - after all components are included - is made up by water.

The aqueous phase of said suspension preferably comprises a pH adjustment agent such that the pH of the suspension 5 or less, preferably 3 or less. The addition of pH adjustment agent also improves microbiological stability.

The aqueous phase may also comprise a food preservative such as a sorbate salt, particularly potassium sorbate. Other additives may be carbohydrates such as glucose syrup, dextrose or sugar, or organic acids, antioxidants and antimicrobials.

The SLNs provided herein may be used in admixtures with one or more additional pigments. Such additional pigments may be in crystalline form. In one particular aspect, a coloring composition may comprise an SLN suspension as defined herein, and a suspension of carotenoid crystals with a mean particle size of below 3 microns stabilized with emulsifiers or hydrocolloids. If the pigment in the SLN suspension defined herein is a carotene the suspension would have a yellow to orange shade. If the crystalline pigment is also a carotene, the crystals will have an orange to reddish shade. By mixing the SLN suspension with the crystals it is possible to yield any orange shade. In a particular embodiment the carotene is a beta-carotene.

The SLNs provided herein may be used in admixtures, in which different SLNs in the same aqueous phase comprise different pigments. In a third aspect, therefore a suspension comprising first solid lipid nanoparticles (SLN-1) and second solid lipid nanoparticles (SLN-2) in a single aqueous phase is provided. Each of SLN-1 and SLN-2 are as defined herein; however, the oil-soluble pigments in each of SLN-1 and SLN-2 are different. Two pigments are termed "different" if their visible absorption spectra differ. In this manner, SLNs with different pigments can be used as building-blocks to provide a desired pigment in admixture.

A food product, an animal feed, a cosmetic or a pharmaceutical product, comprising solid lipid nanoparticles as defined herein is also provided. The SLNs of this technology are particularly suited to liquid food products, preferably beverages.

A further aspect of the present technology is the use of solid lipid nanoparticles as described herein as a colorant in a food product (such as a beverage), an animal feed, a cosmetic or a pharmaceutical product.

A method for producing a suspension of solid lipid nanoparticles (SLNs) is also provided. The method comprises the general steps of:
A. Providing a liquid oil phase comprising (a) a lipid having a melting point above 40°C; and (b) an oil-soluble pigment; and heating said liquid oil phase so as to dissolve the oil-soluble pigment
B. Providing an aqueous phase comprising a dual surfactant system (c), which comprises (i) a polysorbate, and (ii) a phospholipid, such that the ratio of polysorbate:phospholipid (i:ii) is between 10:1 - 1:10;
C. Mixing said liquid oil phase from step A. with said aqueous phase from step B. in a high shear mixer to create an emulsion;
D. Passing the emulsified mixture from step C through a homogenizer;
E. Cooling the homogenized mixture from step D, thereby providing a suspension of solid lipid nanoparticles (SLNs).

### EXAMPLES

### Method for measuring particle size:

The lipid particle size was determined using dynamic light scattering (Zetasizer Nano ZS, Malvern Instruments Ltd, UK) using 10 mm polystyrene cuvettes. Calculations were conducted based on a refractive index of 1.59. The Z-average (Z-ave) refers to average particle size and the PDI refers to the polydispersity index.

### Method for determination of content of beta-carotene:

The content of β-carotene in the particles was determined as follows: The mixture was diluted with water to a β-carotene concentration around 0.06 mg/mL. This solution was then diluted by a factor 50 in acetone. The sample concentration was measured using a conventional Vis spectrophotometer (VWF V-3000PC). The content of β-carotene was calculated using the extension coefficient, E^{1%}_{1cm}, 2559 at 454 nm

### Example 1:

In beaker A, 80.0 g of fully hydrogenated sunflower oil and 5.0 g α-tocopherol was mixed and heated to 165°C. 15.0 g crystalline β-carotene was added and dissolved. In beaker B, 40.0 g of polysorbate 80 and 10.0 g of sunflower lecithin were dissolved in 348.0 g demineralized water and heated to 80°C. Beaker A and B were mixed using a high-shear mixer at 20.000 RPM. The mixture was then passed through a two-valve homogenizer 6 times at a pressure of 800 bar while maintained at 80°C. The homogenized mixture was then cooled to 25°C under stirring (150 rpm). 0.50 g potassium sorbate was added and pH was regulated below 3.00 with citric acid.

Particle size was determined using dynamic light scattering. Z-ave = 144.9 nm, PDI = 0.183. The particle size was stable over a two-month period.

**Table 1 - Particle size over time for example 1**

| Day 0 | | After 2 weeks | | After 1 month | | After 2 months | |
|---|---|---|---|---|---|---|---|
| Z-ave | PDI | Z-ave | PDI | Z-ave | PDI | Z-ave | PDI |
| 144.9 nm ±0.557 nm | 0.183 | 144.1 nm ±1.17 nm | 0.191 | 146.1 nm ±1.78 | 0.207 | 144.5 nm ±1.18 nm | 0.200 |

Concentration was determined to be 2.41 %w/w.

### Example 2:

In beaker A, 70.0 g of fully hydrogenated sunflower oil and 5.0 g α-tocopherol was mixed and heated to 165°C. 25.0 g crystalline β-carotene was added and dissolved. In beaker B, 40.0 g of polysorbate 80 and 10.0 g of sunflower lecithin were dissolved in 348.0 g demineralized water and heated to 80°C. Beaker A and B were mixed using a high-shear mixer at 20.000 RPM. The mixture was then passed through a two-valve homogenizer 6 times at a pressure of 800 bar while maintained at 80°C. The homogenized mixture was then cooled to 25°C under stirring (150 rpm). 0.50 g potassium sorbate was added and pH was regulated below 3.00 with citric acid.

Particle size was determined using dynamic light scattering. Z-average = 167.6 nm, PDI = 0.212. The particle size was stable over a two-month period.

**Table 2 - Particle size over time for example 2**

| Day 0 | | After 2 weeks | | After 1 month | | After 2 months | |
|---|---|---|---|---|---|---|---|
| Z-ave | PDI | Z-ave | PDI | Z-ave | PDI | Z-ave | PDI |
| 167.6 nm ±0.520 nm | 0.212 | 170.5 nm ±1.92 nm | 0.221 | 174.3 nm ±3.48 nm | 0.206 | 169.7 nm ±0.950 nm | 0.221 |

The beta-carotene concentration was determined to be 4.30% w/w.

### Example 3:

In beaker A, 90.0 g of fully hydrogenated sunflower oil and 5.0 g α-tocopherol was mixed and heated to 165°C. 5.0 g crystalline β-carotene was added and dissolved. In beaker B, 40.0 g of polysorbate 80 and 10.0 g of sunflower lecithin were dissolved in 348.0 g demineralized water and heated to 80°C. Beaker A and B were mixed using a high-shear mixer at 20.000 RPM. The mixture was then passed through a two-valve homogenizer 6 times at a pressure of 800 bar while maintained at 80°C. The homogenized mixture was then cooled to 25°C under stirring (150 rpm). 0.50 g potassium sorbate was added and pH was regulated below 3.00 with citric acid.

Particle size was determined using dynamic light scattering. Z-ave = 122.8 nm, PDI = 0.171. The particle size was stable over a two-month period.

**Table 3 - Particle size over time for example 3**

| Day 0 | | After 2 weeks | | After 1 month | | After 2 months | |
|---|---|---|---|---|---|---|---|
| Z-ave | PDI | Z-ave | PDI | Z-ave | PDI | Z-ave | PDI |
| 122.8 nm ±0.702 nm | 0.171 | 124.0 nm ±0.560 nm | 0.150 | 123.6 nm ±1.60 nm | 0.168 | 123.6 nm ±1.27 nm | 0.148 |

The beta-carotene concentration was determined to be 0.92 % w/w.

### Example 4:

In beaker A, 80.0 g of fully hydrogenated sunflower oil and 5.0 g α-tocopherol was mixed and heated to 165°C. 15.0 g crystalline β-carotene was added and dissolved. In beaker B, 25.0 g of polysorbate 80 and 25.0 g of sunflower lecithin were dissolved in 348.0 g demineralized water and heated to 80°C. Beaker A and B were mixed using a high-shear mixer at 20.000 RPM. The mixture was then passed through a two-valve homogenizer 6 times at a pressure of 800 bar while maintained at 80°C. The homogenized mixture was then cooled to 25°C under stirring (150 rpm). 0.50 g potassium sorbate was added and pH was regulated below 3.00 with citric acid.

Particle size was determined using dynamic light scattering. Z-ave = 153.3 nm ± 1.65, PDI = 0.192.

Concentration was determined to be 2.84% w/w.

### Example 5:

In beaker A, 92.5 g of fully hydrogenated sunflower oil and 5.0 g α-tocopherol was mixed and heated to 165°C. 2.50 g crystalline β-carotene was added and dissolved. In beaker B, 40.0 g of polysorbate 80 and 10.0 g of sunflower lecithin were dissolved in 348.0 g demineralized water and heated to 80°C. Beaker A and B were mixed using a high-shear mixer at 20.000 RPM. The mixture was then passed through a two-valve homogenizer 6 times at a pressure of 800 bar while maintained at 80°C. The homogenized mixture was then cooled to 25°C under stirring (150 rpm). 0.50 g potassium sorbate was added and pH was regulated below 3.00 with citric acid.

Particle size was determined using dynamic light scattering. Z-ave = 119.9 nm, PDI = 0.150. The particle size was stable over a two-month period.

**Table 4 - Particle size over time for example 5**

| Day 0 | | After 2 weeks | | After 1 month | | After 2 months | |
|---|---|---|---|---|---|---|---|
| Z-ave | PDI | Z-ave | PDI | Z-ave | PDI | Z-ave | PDI |
| 119.9 nm ±0.681 nm | 0.150 | 121.0 nm ±0.660 nm | 0.143 | 123.5 nm ±1.27 nm | 0.140 | 119.9 nm ±0.839 nm | 0.156 |

Concentration was determined to be 0.449% w/w.

### Example of application:

The product from example 1 was tested in a soft drink medium. Product was dispersed in soft-drink medium to a total β-carotene concentration of approximately 0.2 g/L. The product was tested for heat stability by exposing the soft-drink to 92°C for 40 seconds. The soft drink was also tested for ring formation in plastic bottles. Test bottles were left to stand or lay for 4 weeks after which they were analyzed for ring formation on the plastic. The product passed both application tests. Table 5 shows the results of the heating test:

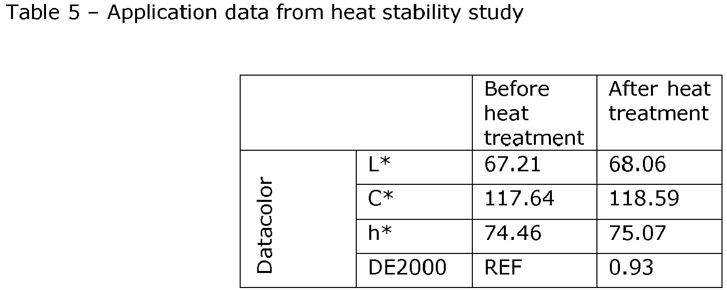

As can be seen from the heat stability study, the products show low color difference (DE2000) before and after heat treatment.

### Comparative example 1:

A preparation was made without adding a phospholipid. In beaker A, 80.0 g of fully hydrogenated sunflower oil and 5.0 g α-tocopherol was mixed and heated to 165°C. 15.0 g crystalline β-carotene was added and dissolved. In beaker B, 50.0 g of polysorbate 80 were dissolved in 348.0 g demineralized water and heated to 80°C. Beaker A and B were mixed using a high-shear mixer at 20.000 RPM. The mixture was then passed through a two-valve homogenizer 6 times at a pressure of 800 bar while maintained at 80°C. The homogenized mixture was then cooled to 25°C under stirring (150 rpm). 0.50 g potassium sorbate was added and pH was regulated below 3.00 with citric acid buffer.

The sample was unstable to moderate shaking/stirring, leading to irreversible agglomeration of the sample. The agglomerated product was not dispersible in water and could as such not be used. Particle size or color could as such not be determined in application.

Although the technology has been described with reference to a number of aspects and examples, the skilled person will be able to combine features from different aspects and examples. Further details of the technology are evident from the appended set of claims.

## Claims

1. A suspension comprising solid lipid nanoparticles (SLNs) as a coloring composition in a food product, wherein the SLN comprise
a. a core comprising:
i. a lipid having a melting point above 40°C, and
ii. an oil-soluble pigment
b. a dual surfactant system comprising
i. a polysorbate, and
ii. a phospholipid
wherein the ratio of polysorbate:phospholipid (i:ii) in said dual surfactant system is between 10:1 and 1:10 and the amount of pigment is 5-40% w/w of said SLN;
wherein said suspension comprises:
iii. a lipid having a melting point above 40°C, in an amount of 10-35% w/w of the total suspension; and
iv. an oil-soluble pigment, in an amount of 1-20% w/w of the total suspension;
v. a dual surfactant system, in an amount of 1-25% w/w of the total suspension.

2. The suspension of claim 1, wherein said SLN additionally comprises an antioxidant, suitably a tocopherol such as α-tocopherol.

3. The suspension according to any one of the preceding claims, wherein said antioxidant is present in an amount of between 1 and 10% w/w of said SLN.

4. The suspension according to any one of the preceding claims, wherein said lipid is present in an amount of between 30 and 70% w/w of said SLN.

5. The suspension according to claim 4, wherein said lipid is a fully saturated triglyceride, such as e.g. fully hydrogenated sunflower oil, fully hydrogenated rapeseed oil fully hydrogenated palm oil or fully hydrogenated soybean oil.

6. The suspension according to any one of the preceding claims, wherein said oil-soluble pigment is a carotenoid or a chlorophyll.

7. The suspension according to claim 6, wherein the carotenoid is a lycopene or a beta-carotene.

8. The suspensions according to any one of the preceding claims, wherein said dual surfactant system is present in an amount of between 20 and 75% w/w of said SLN.

9. The suspension according to any one of the preceding claims, wherein said polysorbate is polysorbate 80.

10. The suspension according to any of the preceding claims, comprising said lipid having a melting point above 40°C in an amount of 5-30% w/w, preferably 10-20% w/w more preferably 14-18 % w/w of the total suspension.

11. The suspension according to any one of the preceding claims, comprising said phospholipid in an amount of 2-10% w/w, preferably 2-5% w/w of the suspension or comprising said polysorbate in an amount of 5-10% w/w.

12. The suspension according to any one of the preceding claims, wherein said oil-soluble pigment is present in an amount of 0.5-20% w/w, preferably 1-5% w/w of said suspension.

13. A suspension comprising first solid lipid nanoparticles (SLN-1) and second solid lipid nanoparticles (SLN-2) in a single aqueous phase, wherein each of SLN-1 and SLN-2 are defined as per any one of the preceding claims, and wherein the oil-soluble pigments in each of SLN-1 and SLN-2 are different.

14. A food product, preferably a beverage, comprising a suspension according to any one of the preceding claims.

15. A method for coloring a food, a beverage, a dietary supplement or a pharmaceutical product with the use of the SLN suspension according to any of claims 1-13.
